# EUROPEAN PATENT APPLICATION

(11) **EP 1 702 637 A1**
(43) Date of publication of application: **20.09.2006**
(21) Application number: 05388022.5
(22) Date of filing: 15.03.2005
(51) Int. Cl.: A61M 5/50

(54) **A disposable and non-reusable syringe**

(71) Applicant: Emunio ApS, 2970 Horsholm (DK)
(72) Inventor: Norgaard, Tina, 2970 Horsholm (DE)
(74) Representative: Nielsen, Henrik Sten

(57) **Abstract**

A disposable and non-reusable syringe (10) comprises a hollow housing component (12), a piston component (14) having a piston head (32) and a piston shaft (34), the piston head (32) and the piston shaft (34) being connected through connection means (50). The piston component (14) is received in the hollow housing component (12) for axial movement therein between a first position and a second position. The hollow housing component (12) and the piston component (14) further include blocking means (36,46) for allowing the piston component (14) to be moved once unidirectionally from the first position to the second position and further to be moved once unidirectionally from the second position towards the first position and arresting the piston head (32) relative to the proximal position when the piston component (14) reaches the first position. The connection means (50) are breakable for causing the piston shaft (34) to be disconnected from the piston head (32) provided an attempt is made to move once again the piston component (14) from the first position towards the second position after the piston head (32) has been arrested by means of the blocking means (36,46). Further, the hollow housing component (12) and the piston component (14) are made from the same polymer material, preferably PP, PE, such as HDPE, MDPE, LDPE or combinations thereof.

## Description

The present invention relates to a disposable and non-reusable syringe, i.e. a syringe which may be used only once and which is of a structure preventing the syringe from being used more than once as the syringe is simply destroyed provided an attempt is made to use the syringe again.

In the literature, a number of examples describe various technical solutions to the problem of providing a disposable and non-reusable syringe. Examples of prior art references describing such syringes: DE 42 34 383, US 2001/041866, US 4,213,456, US 2004/0059300, FR 2 638 645, US 5,222,942, DE 40 34 673, WO 03/037411, US 6, 533,756, US 5.562,623, US 5,531,691, and WO 90/03818. Reference is made to the above patent applications and patens, and the above US publications are hereby incorporated in the present specification by reference.

In the third world, a major problem exists in relation to the dispensing of medication to patients since unfortunately in many situations, a medical dispensing syringe is used for dispensing more than a single dose to a single patient which inevitably cause contamination of serious diseases such as bacterial and virus based diseases, hepatitis and aids.

The above publications all claim to have solved the problems of preventing the disposable syringe from being used more than once, however, the commercially available structures all suffer from one drawback in that the commercially available disposable and non-reusable syringes cannot easily be disposed without risk to the person or the persons serving to dispose the disposable syringes, mainly for the reason that the commercial available disposable and non-reusable syringes include components of different materials such as plastics material barrels, plastics material pistons, O-ceiling rings, metallic cutting or blocking elements, etc.

Provided the disposable syringes are to be disposed in the third world, the syringes are often simply combusted, however, the presence of the various different materials cause a pollutional problem provided the disposable syringes by burned as the varying materials have different combustion temperatures and therefore cause the generation of toxic gasses.

Furthermore, the presence of metallic components in the disposable syringes cause a waste problem and, generally speaking, the presence of different materials in the various components of the disposable and non-reusable syringes commercially available only allows reuse of the materials provided a disassembling and disintegration of the various components of the syringes be made, which of course is entirely unacceptable from the point of view of the health of the persons serving to dispose the syringes.

An object of the present invention is to provide a reliable and safe disposable and non-reusable syringe, i.e. a syringe which cannot be tampered with and consequently can only be manipulated and used once without any risk of having the syringe used more than once or having the syringe used partially by disposing only a fraction of the medical dose and then reloading the syringe which unfortunately is the case with many commercially available so-called non-reusable syringes.

A further object of the present invention is to provide a disposable and non-reusable syringe which may be easily disposed or reused without any risk of causing contamination or pollution provided the syringe be burned or in the alternative allows the syringe to be easily recycled by reusing the plastics materials of the syringe.

A particular features of the present invention relates to the fact that the disposable and non-reusable syringe is of a structure in which the integral hollow needle is easily separable from the plastics material barrel of the syringe by breaking off a minor part of the end part of the barrel which end part includes the hollow metallic needle and, consequently provides a residual syringe including only the plastics material of the disposable and non-reusable syringe without any metallic components.

The above objects, the above feature together with other objects, features and advantages which will be evident to a person having ordinary skill in the art is, according to the teachings of the present invention obtained by a disposable and non-reusable syringe comprising:
a piston component having a piston head and a piston shaft, the piston head and the piston shaft being connected through connection means, the piston component being received in the housing component for axial movement therein between a first position in which the piston head is positioned juxtaposed the proximal end and a second position in which the piston head is spaced from the proximal end,
the housing component and the piston component further including blocking means for allowing the piston component to be moved once unidirectionally from the first position to the second position and further to be moved once unidirectionally from the second position towards the first position and arresting the piston head relative to the proximal position when the piston component reaches the first position,
the connection means being breakable for causing the piston shaft to be disconnected from the piston head provided an attempt is made to move once again the piston component from the first position towards the second position after the piston head be arrested by means of the blocking means, and
the hollow housing component and the piston component being made from the same polymer material, preferably PP, PE, such as HDPE, MDPE, LDPE or combinations thereof.

A feature characteristic of the present invention relates to the fact that the housing component and the piston component are both made from a plastics or polymer materials, preferably PP or in the alternative PE, without the use of any additional elements such as metallic blocking elements or ceiling components made from e.g. a different kind of elastomer or rubber material.

According to a particular feature of the disposable and non-reusable syringe according to the present invention, the hollow needle is easily separable from the hollow housing component by breaking off the hollow needle at a weakening position.

The piston component is preferably made as a unitary structure, although of course the present component may alternatively be composed of two separate components constituting the piston head and the piston shaft. According to the presently preferred embodiment of the disposable and non-reusable syringe according to the present invention, the blocking means and the piston head and the piston shaft are all manufactured in a single component which prior use of the disposable and non-reusable syringe are separated into the piston component and the blocking means as the syringe is prepared for use by forcing the present component initially including integrally the blocking means into the hollow housing component and in doing so loading or preparing the syringe for use by separating the blocking means from the piston component.

According to the above described preferred embodiment of the disposable and non-reusable syringe according to the present invention, the blocking means include a shiftable part shiftable from a first state in which the piston component is freely movable from the first position to the second position, and to a second state when initiating the movement of the piston from the second position towards the first position for causing the unidirectional movement from the second position towards the first position. The shiftable part is preferably constituted by a part circumferential enclosing a ratchet shaft of the piston component and initially carried along with the piston component as the piston component is moved from the first position to the second position and due to its friction with the hollow housing component prevented from being moved in the reverse direction as any attempt to move the piston in the reverse direction causes unilateral motion of the piston component relative to the blocking means due to the present of the ratchet mechanism included in the cooperating blocking means and piston component.

The invention is now to be further described with reference to the drawings in which
Fig. 1a is a vertical sectional view of a hollow housing component of a preferred embodiment of a disposable and non-reuseable syringe according tot the present invention,
Fig, 1b is a side elevational view of a three-part piston to be introduced into the hollow housing component shown in Fig. 1a of the preferred embodiment of the disposable and non-reusable according to the present invention,
Fig. 1c is a side elevational view of the piston also shown in Fig. 1 b after assembling the three-part piston,
Fig. 1d is a perspective view of the piston shown in Fig. 1 b before assembling the three-part piston as illustrated in Fig. 1c,
Fig. 2 is a partly vertical sectional view similar to the view of Fig. 1 a illustrating the hollow housing component of the preferred embodiment of the disposable non-reusable syringe and illustrating the piston prior to use of the piston,
Fig. 3 is a view similar to the view of Fig. 2 illustrating the piston ready to be used,
Fig. 4 is a view similar to the views of Figs. 2 and 3 illustrating a first step of use of the piston, and
Fig. 5 is a view similar to the views of Figs. 2, 3 and 4 illustrating a second step of use of the piston.

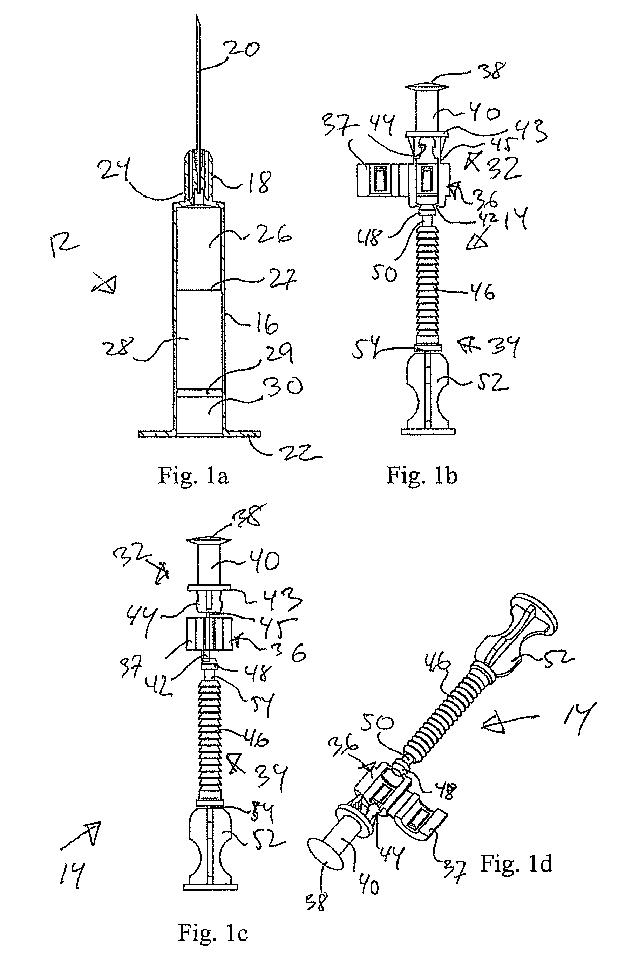

In Fig. 1a, a hollow housing component according to a preferred embodiment of a disposable and non-reusable syringe according to the present invention is shown designated the reference numeral 12.

In Fig. 1b, a three-part piston of the preferred embodiment of the disposable and non-reusable syringe according to the present invention is shown designated the reference numeral 14.

The hollow housing component 12 generally comprises a cylindrical barrel 16 defining a proximal end and a distal end. At the proximal end, the cylindrical barrel 16 is narrowed into a reduced diameter end part 18 in which a hollow needle 20 is fixated. At the distal end, the cylindrical barrel 16 is provided with a circumferential gripping flange 22. A particular feature of the hollow housing component 12 is the provision of a breaking generator 24 constituted by a circumferential cut defining a weakening zone allowing the hollow needle 20 to be easily separated from the remaining part of the hollow housing component after the use by breaking off the front part of the reduced diameter front part of the hollow housing component along the weakening zone defined by the circumferential cut 24.

The cylindrical barrel 16 of the hollow housing component 12 defines three sections, a proximal section 26, a central section 28 and a distant section 30. The proximal section 26 has the minimum inner diameter of all three sections, such as a diameter of 8.00 mm. A line 27 defines the transition from the proximal section 26 to the central section 28 which section has a larger inner diameter as compared to the proximal section 26, such as a diameter of 8.30 mm. A transition section 29 between the central section 28 and the distal section 30 of the cylindrical barrel 16 of the hollow housing component 12 is designated the reference numeral 29 and has an inner diameter slightly longer than the inner diameter of the proximal section 26, however, smaller than the inner diameter of the central section 28 and also smaller than the inner diameter of the distal section 30, such as a diameter of 8.10 mm. The distal section 30 defines the major inner diameter of all three sections of the cylindrical barrel 16, such as a diameter of 8.40 mm. The above figures serve as examples for defining the functionality of the disposable and non-reusable syringe which syringe advantageously and preferably has an inner volume of the order of 0.5-0.7 ml, i.e. an inner volume for dispensing a volume of the order of 0.5-0.7 ml. The overall length of the hollow housing component 12 without the hollow needle 20 may e.g. be 65 mm providing the inner volume is 0.5-0.7 ml.

In Fig. 1b, the three-part piston 14 of the disposable and non-reusable syringe is shown. The three-part piston 14 comprises a stem part 32, a shaft part 34 and a blicking part 36. For manufacturing purposes, the blocking part 36 is advantageously cast in the open state as is illustrated in Fig. 1b, as the blocking part 36 is simply closed by closing the one half of the blocking part 36, which one half is designated the reference numeral 37, for converting the open blocking part 36 shown in Fig. 1 b into a closed and ready to use state as is illustrated in Fig. 1c. Initially, i.e. in the pre-use state, the stem part 32 is linked to the open blocking part 36 through breakable pins 45, and the open blocking part 36 is also connected through additional breakable pins 42 to the shaft part 34.

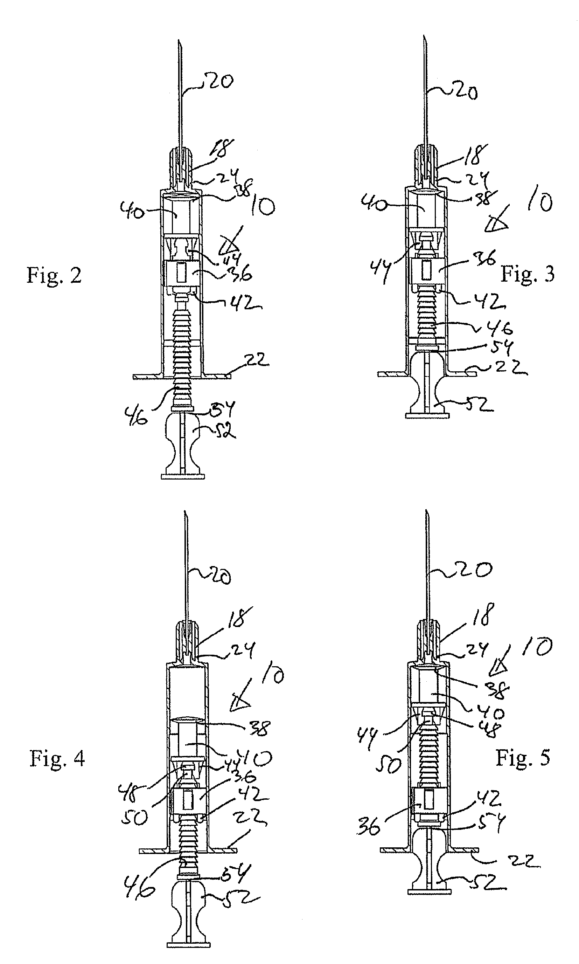

The stem part 32 comprises at its front end a front plate 38 which is fixated to a shaft receiving part 43 by means of a cylindrical stem 40. The shaft receiving part 43 has three gripping claws 44 configurated so as to allow the claws to grip around and arrest a front end or head 48 of the shaft part 34, as will be described in greater details below with reference to Figs. 2 and 3.

The shaft 34 has a central ratchet part 46 and a shaft front end including the above mentioned head 48 connected to the ratchet part 46 through a reduced diameter cylindrical part 50. At the rear end of the shaft 34, a pressure head 52 is connected to the ratchet part 46 through a reduced diameter and weakened part 54 which is easily broken provided the head 52 is pulled backwards as will be described below with reference to Fig. 5.

In Fig. 1c, the three-part piston 14 is shown in its ready-to-use state as discussed above, and in Fig. 1d, the open three-part piston 14 is shown in a perspective view disclosing in greater details the opened and ready-to-close part 36 of the three-part piston 14.

In Fig. 2, the preferred embodiment of the disposable and non-reusable syringe of the present invention is shown. In Figs. 2, 3, 4 and 5, the disposable and non-reusable syringe is in its entirety designated the reference numeral 10. In Fig. 2, a step of preparing the syringe 109 for use is shown, as the stem part 32 is introduced into a cylindrical barrel 16 of the hollow housing component 12 after the closing of the blocking part 36 and forced forward to the proximal end of the hollow housing component 12. In the process of forcing the three-part piston 14 from the distal end to the proximal end of the cylindrical barrel 16 of the hollow housing component 12, the blocking part 36 is slightly compressed or deformed for allowing the blocking part 36 to be forced past the constriction 29 and is stopped at the transition 27 between the proximal section 26 and the central section 28 of the cylindrical barrel 16 of the hollow housing component 12. In the position shown in Fig. 2, the blocking component 36 is resting against the transition 27 and the head 38 is positioned a short distance from the proximal end wall of the cylindrical barrel 16 of the hollow housing component 12. By applying pressure to the head 52 of the shaft part 34, the head 48 breaks the breakable pins 42, punches into the head receiving part 43 of the stem part 32 and in doing so breaks off the stem part 32 from the blocking part 36 by breaking the breakable pins 45 as is illustrated in Fig. 3 and presses the head 48 of the stem part 32 into contact with the proximal end wall of the cylindrical barrel 16 of the hollow housing component 12.

The syringe 10 is now ready for use. From the position shown in Fig. 3, the piston 14 is retracted from its proximal position shown in Fig. 3 to its distal position in which the blocking component 36 locks against the constriction 29 at the transition between the central section 28 and the distal section 30 of the cylindrical barrel 16 of the hollow housing component 12.

From the position shown in Fig. 4, the stem 14 is forced through the blocking part 36 as the ratchet part 46 of the shaft part 34 of the piston 14 is forced through the blocking part 36 in which the previously broken pins 45 are folded inwardly and serve to cooperate with the individual ratchets of the ratchet part 46 of the shaft component 34 preventing the piston 14 from being retracted once again. It is to be understood that in use of the disposable and non-reusable syringe 10, the step from Fig. 3 to Fig. 4 is used for introducing through suction a medical dispension into the proximal section 26 of the cylindrical barrell 16 which medical suspension is then forced out from the proximal section 26 as the piston as shown in Fig, 5 is forced to its proximal position as the ratchets of the ratchet part 46 of the shaft part 14 of the piston is forced through the blocking part 36.

Provided an attempt is made to pull the stem backwards from the position shown in Fig. 5, the head 52 is broken off at the reduced diameter and weakened part 54 as the force needed for breaking off the head 52 is far smaller than the force needed for forcing the ratchet part 46 in the non-intentional direction through the blocking part 36.

After use, the metallic hollow needle 24 is broken off along the weakening line 24 and the remaining and major part of the disposable and non-reusable syringe is then ready to be combusted or reused since the entire syringe is made from the same material, viz. a plastics or polymer material such as PE, e.g. HDPE, MDPE, LDPE or a combination thereof or preferably and advantageously PP.

It is to be understood that the steps of preparing the syringe 10 for use, i.e. the steps illustrated in Figs. 2 and 3, are carried out before the syringe leaves the factory since the syringe as shipped for use has to be loaded and prepared for only one retraction of the piston for loading the medical dose into the inner volume of the syringe, and only one appending forward motion of the piston for discharging the medical dose.

Although the present invention has above been described with reference to a specific and presently preferred embodiment of the disposable and non-reusable syringe according to the present invention, it is to be understood that numerous modifications may be embodied in a variant of the above described embodiment without departing from the spirit and scope of the invention as defined in the appending claims.

## Claims

1. A disposable and non-reusable syringe comprising:
a piston component having a piston head and a piston shaft, said piston head and said piston shaft being connected through connection means, said piston component being received in said hollow housing component for axial movement therein between a first position in which said piston head is positioned juxtaposed said proximal end and a second position in which said piston head is spaced from said proximal end,
said hollow housing component and said piston component further including blocking means for allowing said piston component to be moved once unidirectionally from said first position to said second position and further to be moved once unidirectionally from said second position towards said first position and arresting said piston head relative to said proximal position when said piston component reaches said first position,
said connection means being breakable for causing said piston shaft to be disconnected from said piston head provided an attempt is made to move once again said piston component from said first position towards said second position after said piston head be arrested by means of said blocking means, and
said hollow housing component and said piston component being made from the same polymer material, preferably PP, PE, such as HDPE, MDPE, LDPE or combinations thereof.

2. The syringe according to claim 1, said hollow needle being separable from said hollow housing component by breaking off said hollow needle at a weakening position.

3. The syringe according to any of the claims 1 or 2, said piston component being a unitary structure.

4. The syringe according to any of the claims 1-3, said blocking means including a shiftable part shiftable from a first state in which said piston component is freely movable from said first position to said second position; to a second state when initiating the movement of said piston component from said second position towards said first position for causing said unidirectional movement from said second position towards said first position.

5. The syringe according to claim 4, said shiftable part and/or said hollow housing component including a ratchet mechanism.
